# EUROPEAN PATENT APPLICATION

(11) **EP 3 733 657 A1**
(43) Date of publication of application: **04.11.2020**
(21) Application number: 18894831.9
(22) Date of filing: 02.07.2018
(51) Int. Cl.: C07D 311/72, C07D 207/16, A61K 31/401, A61P 39/06, A23K 20/142, A23K 20/121, A23K 20/174, A23L 33/175, A23L 33/15

(54) **COCRYSTAL OF TOCOPHEROL AND PROLINE AND PREPARATION METHOD THEREFOR**

(30) Priority: 26.12.2017 CN 201711435511
(71) Applicant: COCRYSTAL PHARMACEUTICAL TECHNOLOGY (SHANGHAI) CO., LTD., Zhangjiang Pudong Shanghai 201203 (CN)
(72) Inventor: MEI, Xuefeng, Shanghai 201203 (CN); ZHU, Bingqing, Shanghai 201203 (CN); WANG, Jianrong, Shanghai 201203 (CN); ZHANG, Qi, Shanghai 201203 (CN)
(74) Representative: Kador & Partner PartG mbB
(86) International application number: PCT/CN2018/093967
(87) International publication number: WO 2019/128175

(57) **Abstract**

The invention relates to a co-crystal of tocopherol and proline and a preparation method thereof. The co-crystal is in a crystalline or partially crystalline form under normal pressure at room temperature. Compared with the existing tocopherol, the co-crystal has a higher melting point, better crystallinity, and has more excellent high-temperature stability, light stability, accelerated stability and long-term stability. The health products, food, cosmetics or feed products prepared by the present co-crystal have significantly improved stability. The present co-crystal maintains the excellent antioxidant property of tocopherol and can be widely used as an antioxidant in the fields of food, medicine and the like. The invention greatly improves the convenience in the use of tocopherol and saves the cost in the process of manufacturing, storage, transportation and use of tocopherol.

## Description

### Technical field

The invention relates to a co-crystal of tocopherol and proline and a preparation method thereof. The co-crystal is in a crystalline or partial crystalline form under normal pressure at room temperature. The co-crystal has better crystallinity, higher tocopherol content, and better thermal stability, light stability, accelerated stability and long-term stability. In the co-crystal, tocopherol and proline interact with each other via non-covalent bonds.

### Background

Tocopherol has anti-oxidation effect, and is widely applied in the fields of medicine, food production, feed industry production, cosmetics, etc. Tocopherols include natural tocopherol and synthetic tocopherol. Natural tocopherol comprises two types of tocopherols and tocotrienols, and comprises 8 kinds of compounds, namely α-, β-, γ-, δ-tocopherols and α-, β-, γ-, δ-tocotrienols. Synthetic tocopherol refers to α-tocopherol, and has 8 optical isomers. Among them, D-α-tocopherol is a form of tocopherol which is most widely distributed in nature, most abundant, and has the highest activity.

However, tocopherol, which is in the form of an oily liquid, is sensitive to oxygen, and unstable when exposed to air, thus causing various problems, such as the inconvenience when weighing during the production, transportation, and use, poor compatibility with excipients, instability, etc. In order to improve the stability of tocopherol, it is used after being derived to tocopherol acetate or succinate, and further converted into a solid powder by embedding techniques. However, the content of tocopherol in the embedded solid powder is relatively low (the highest content is 50% for the commercially available tocopherol), and the antioxidant property of tocopherol is closely related to the phenolic hydroxyl group in its chemical structure. The esterified tocopherol loses its antioxidant property, limiting its application as an antioxidant. Therefore, it is constant pursuit to obtain a tocopherol product that is convenient to use, and has high content of tocopherol, high chemical stability and excellent antioxidant property. In the Japanese Patent JP2008024603(A), a complex of tocopherol itself and proline was prepared by a distillation method under reduced pressure. Although this complex is in a solid state, the tocopherol and proline in the complex are mostly amorphous, and the stoichiometric ratio therebetween is not fixed, and it shows a poor powder state.

### Summary of the invention

In the present invention, the term "co-crystal" refers to a chemical substance in which there is a covalent bond(s) in the two compounds and the two compounds (tocopherol and proline) interact with each other via the non-covalent bond(s). Generally, hydrogen bonds and other types of bonds, such as van der Waals forces, can be observed between the two compounds. These interactions cause the two compounds present in the co-crystal to combine with each other. Such combination distinguishes the co-crystal from the physical mixture of these two compounds in their properties (such as melting point, IR spectrum, etc.).

It is well known that a crystalline form of a compound generally has a higher melting point and better stability. After researches, the present inventor has found that tocopherol can form co-crystals with proline. The co-crystal formed has the following significant advantages: 1. Compared with the tocopherol complex, the co-crystal has better crystallinity and increased melting point; 2. Compared with the tocopherol itself, the co-crystal has significantly improved stability; 3. Compared with semi-synthetic or fully synthetic tocopherol, the co-crystal has more excellent antioxidant effect. Therefore, the co-crystal of tocopherol and proline has good application value in practice.

In a first aspect, the present invention provides a co-crystal of tocopherol and proline.

Preferably, the proline is D-proline, or L-proline, or a racemic mixture of the both. The molecular structure thereof is as follows:

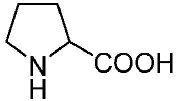

Preferably, the proline is L-proline.

In the present invention, the tocopherol is selected from one or more of natural tocopherol and synthetic tocopherol.

The natural tocopherol comprises α-, β-, γ-, δ-tocopherols and α-, β-, γ-, δ-tocotrienols, and the stereo configuration of C2, C4' and C8' is RRR. The molecular structure of natural tocopherol is shown below:

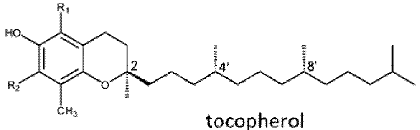

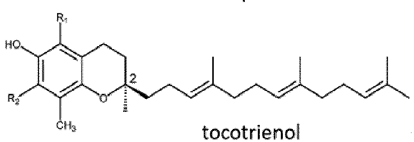

| Name of natural tocopherol | R₁ | R₂ |
|---|---|---|
| α-tocopherol/α-tocotrienol | CH₃ | CH₃ |
| β-tocopherol/β-tocotrienol | CH₃ | H |
| γ-tocopherol/γ-tocotrienol | H | CH₃ |
| δ-tocopherol/δ-tocotrienol | H | H |

The synthetic tocopherol is an equimolar mixture of eight optical isomers of DL-α-tocopherol. The C2, C4' and C8' stereo configurations of the eight optical isomers are RRR, SSS, RSR, SRS, RRS, SSR, SRR and RSS, respectively. The molecular structure thereof is shown below,

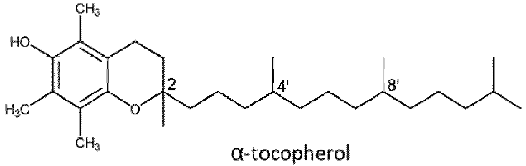

Preferably, the tocopherol is D-α-tocopherol.

In a second aspect, the present invention further provides a method for preparing the co-crystal of tocopherol and proline, which comprises the following steps:
(i) Dissolving tocopherol and proline in a suitable solvent and stirring the same at 10-80°C until they are dissolved;
(ii) Recrystallizing the solution obtained from step (i);
(iii) Separating the solid and drying the same.

In a preferred embodiment of the present invention, in step (i), the suitable solvent is a common organic solvent selected from but not limited to water, methanol, ethanol, isopropanol, acetone, methyl ethyl ketone, acetonitrile, tetrahydrofuran, nitromethane, ethyl acetate, isopropyl acetate, methyl tert-butyl ether, toluene, DMF and methylene chloride and the like, and a mixed solvent thereof; in a more preferred embodiment of the present invention, in step (i), the suitable solvent is selected from but not limited to methanol, ethanol, nitromethane, acetone and methyl ethyl ketone;

The co-crystal of tocopherol and proline according to the invention is in a crystalline or partial crystalline form under normal pressure at room temperature. The stoichiometric ratio of tocopherol to proline in the co-crystal is 1:2, 1:1 or 2:1.

In a preferred embodiment of the present invention, the co-crystal is a co-crystal formed by D-α-tocopherol and L-proline.

Wherein, the stoichiometric ratio of D-α-tocopherol to L-proline in the co-crystal is 1:2-2:1, preferably 1:2, 1:1 or 2:1.

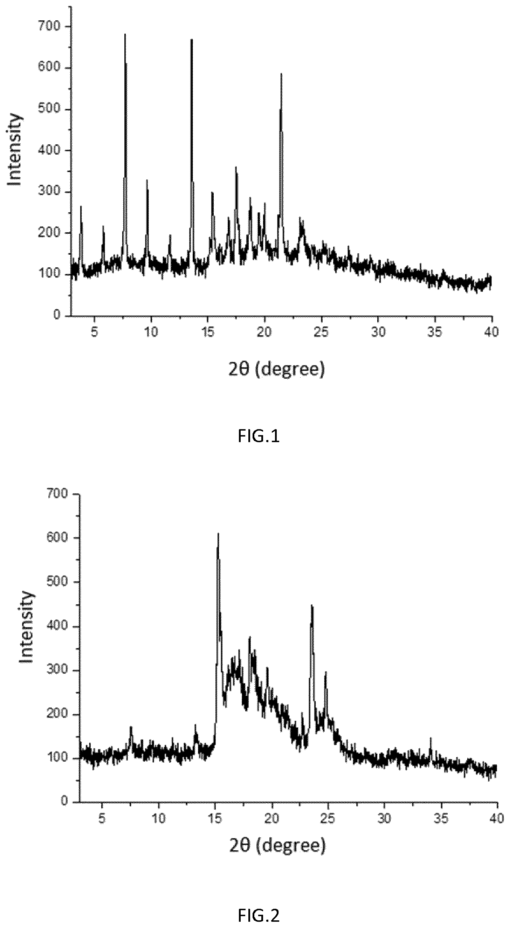

Provided is a co-crystal of D-α-tocopherol and L-proline. The co-crystal is in a crystalline form under normal pressure at room temperature. The stoichiometric ratio of D-α-tocopherol to L-proline in the co-crystal is 1:1. It is characterized by an X-ray powder diffraction pattern having characteristic peaks at 2θ diffraction angle of 3.8±0.2, 5.8±0.2, 7.7±0.2, 9.7±0.2, 11.6±0.2, 13.6±0.2, 15.4±0.2, 17.5±0.2, 21.4±0.2 degrees; preferably, the X-ray powder diffraction pattern further has characteristic peaks at 2θ diffraction angle of 16.8±0.2, 18.8±0.2, 19.5±0.2, 23.4±0.2 degrees; more preferably, it has an X-ray powder diffraction pattern shown in FIG. 1.

The preparation method for the above co-crystal is as follows: D-α-tocopherol and L-proline are added at a stoichiometric ratio of 1:1 to a mixed solvent of methanol and nitromethane (preferably, the volume ratio of methanol to nitromethane is 1:1), and the resultant is stirred at 20-60°C for 0.5-2 hours, and recrystallized to obtain a white precipitate. The precipitate is filtered and the solid thus obtained is dried at room temperature to give a co-crystal of D-α-tocopherol and L-proline.

Preferably, the preparation method for the above co-crystal is as follows: D-α-tocopherol (5 mmol) and L-proline (5 mmol) are added at a stoichiometric ratio of 1:1 to 20 ml of a mixed solvent of methanol and nitromethane (volume ratio 1: 1), and the resultant is stirred at 40°C for 1 hour, and recrystallized to obtain a white precipitate. The precipitate is filtered through a Buchner funnel, and the solid is dried in a vacuum oven at room temperature for 1 day to give a co-crystal of D-α-tocopherol and L-proline.

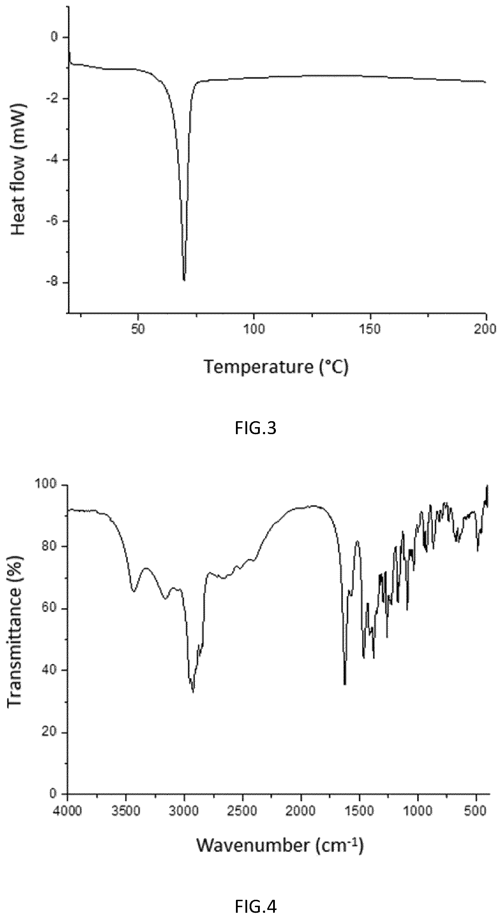

The 1:1 co-crystal formed by D-α-tocopherol and L-proline is determined by differential scanning calorimetry, the melting initiation temperature is 66±3°C, and the maximum peak value is 70°C; preferably, it has a differential scanning calorimetry thermogram shown in FIG. 3.

The 1:1 co-crystal formed by D-α-tocopherol and L-proline is characterized by the infrared absorption spectrum obtained by using a Fourier transform infrared spectrometer, and is characterized by the following important wave numbers: 3438 cm⁻¹, 3163 cm⁻¹, 2926 cm⁻¹, 1626 cm⁻¹, 1564 cm⁻¹, 1462 cm⁻¹, 1377 cm⁻¹, 1294 cm⁻¹, 1262 cm⁻¹, 1171 cm⁻¹, 1089 cm⁻¹, 1034 cm⁻¹, 923 cm⁻¹, 864 cm⁻¹, 670 cm⁻¹, 484 cm⁻¹; preferably, it has an infrared spectrum shown in FIG. 4.

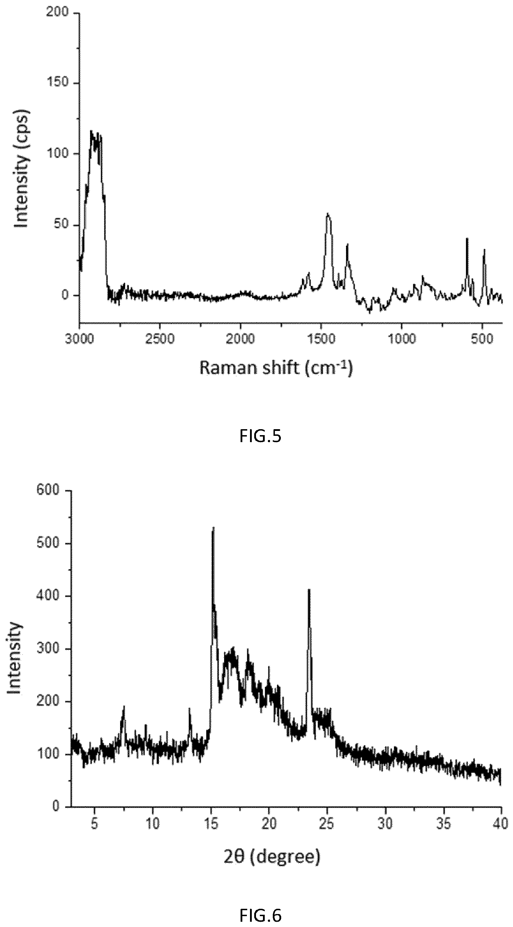

The 1:1 co-crystal formed by D-α-tocopherol and L-proline is characterized by the Raman spectrum obtained by using a laser Raman spectrometer, and is characterized by the following important wave numbers: 2922 cm⁻¹, 2870 cm⁻¹, 1617 cm⁻¹, 1580 cm⁻¹, 1464 cm⁻¹, 1393 cm⁻¹, 1340 cm⁻¹, 596 cm⁻¹, 566 cm⁻¹, 491 cm⁻¹; preferably, it has a Raman spectrum shown in FIG. 5.

Provided is a co-crystal of D-α-tocopherol and L-proline. The co-crystal is in a partial crystalline form under normal pressure at room temperature. The stoichiometric ratio of D-α-tocopherol to L-proline in the co-crystal is 1:1. It is characterized by an X-ray powder diffraction pattern having characteristic peaks at 2θ diffraction angle of 7.6±0.2, 13.2±0.2, 15.3±0.2, 18.1±0.2, 19.5±0.2, 23.5±0.2, 24.8±0.2 degrees; preferably, it has an X-ray powder diffraction pattern shown in FIG. 2.

The preparation method for the above co-crystal is as follows: D-α-tocopherol and L-proline are added at a stoichiometric ratio of 1:1 to methanol solvent, and the resultant is stirred at 20-60°C for 0.5-2 hours, and recrystallized to obtain a white precipitate. The precipitate is filtered and the solid thus obtained is dried at room temperature to give a co-crystal of D-α-tocopherol and L-proline.

Preferably, the preparation method for the above co-crystal is as follows: D-α-tocopherol (5 mmol) and L-proline (5 mmol) are added at a stoichiometric ratio of 1:1 to 10 ml of methanol solvent, and the resultant is stirred at 40°C for 1 hour, and recrystallized to obtain a white precipitate. The precipitate is filtered through a Buchner funnel, and the solid is dried in a vacuum oven at room temperature for 1 day to give a co-crystal of D-α-tocopherol and L-proline.

The 1:1 co-crystal formed by D-α-tocopherol and L-proline is determined by differential scanning calorimetry, the melting initiation temperature is 66±3°C, and the maximum peak value is 70°C; preferably, it has a differential scanning calorimetry thermogram shown in FIG. 3.

The 1:1 co-crystal formed by D-α-tocopherol and L-proline is characterized by the infrared absorption spectrum obtained by using a Fourier transform infrared spectrometer, and is characterized by the following important wave numbers: 3438 cm⁻¹, 3163 cm⁻¹, 2926 cm⁻¹, 1626 cm⁻¹, 1564 cm⁻¹, 1462 cm⁻¹, 1377 cm⁻¹, 1294 cm⁻¹, 1262 cm⁻¹, 1171 cm⁻¹, 1089 cm⁻¹, 1034 cm⁻¹, 923 cm⁻¹, 864 cm⁻¹, 670 cm⁻¹, 484 cm⁻¹; preferably, it has an infrared spectrum shown in FIG. 4.

The 1:1 co-crystal formed by D-α-tocopherol and L-proline is characterized by the Raman spectrum obtained by using a laser Raman spectrometer, and is characterized by the following important wave numbers: 2922 cm⁻¹, 2870 cm⁻¹, 1617 cm⁻¹, 1580 cm⁻¹, 1464 cm⁻¹, 1393 cm⁻¹, 1340 cm⁻¹, 596 cm⁻¹, 566 cm⁻¹, 491 cm⁻¹; preferably, it has a Raman spectrum shown in FIG. 5.

The present invention also provides another co-crystal of D-α-tocopherol and L-proline. The co-crystal is in a partial crystalline form under normal pressure at room temperature. The stoichiometric ratio of D-α-tocopherol to L-proline in the co-crystal is 2:1. It is characterized by an X-ray powder diffraction pattern having characteristic peaks at 2θ diffraction angle of 7.5±0.2, 13.2±0.2, 15.2±0.2, 23.4±0.2 degrees; preferably, the X-ray powder diffraction pattern further has characteristic peaks at 2θ diffraction angle of 9.4±0.2, 18.6±0.2, 19.9±0.2 degrees; more preferably, it has an X-ray powder diffraction pattern shown in FIG. 6.

The preparation method for the above co-crystal is as follows: D-α-tocopherol and L-proline are added at a stoichiometric ratio of 2:1 to methanol solvent, and the resultant is stirred at 20-60°C for 0.5-2 hours, and recrystallized to obtain a white precipitate. The precipitate is filtered and the solid thus obtained is dried at room temperature to give a co-crystal of D-α-tocopherol and L-proline.

Preferably, the preparation method for the above co-crystal is as follows: D-α-tocopherol (10 mmol) and L-proline (5 mmol) are added at a stoichiometric ratio of 2:1 to 10 ml of methanol solvent, and the resultant is stirred at 40°C for 1 hour, and recrystallized to obtain a white precipitate. The precipitate is filtered through a Buchner funnel, and the solid is dried in a vacuum oven at room temperature for 1 day to give a co-crystal of D-α-tocopherol and L-proline.

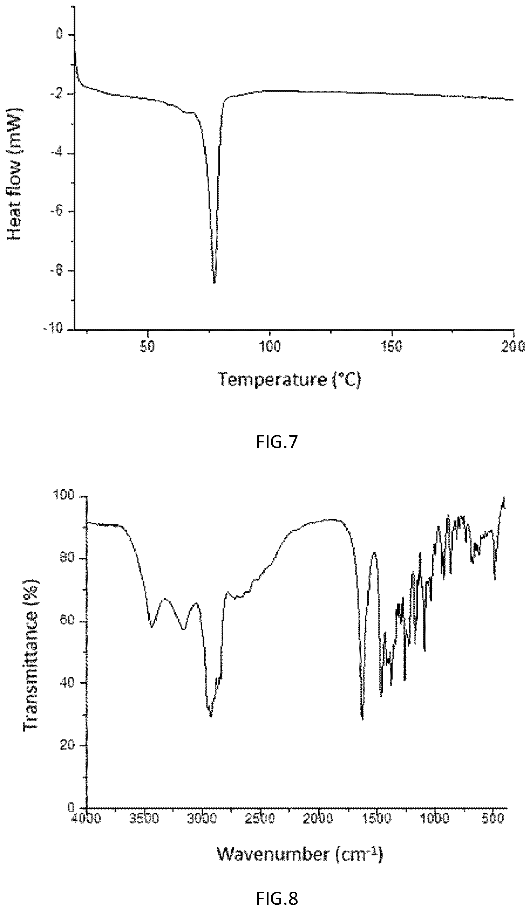

The 2:1 co-crystal formed by D-α-tocopherol and L-proline is determined by differential scanning calorimetry, the melting initiation temperature is 73±5°C, and the maximum peak value is 77°C; preferably, it has a differential scanning calorimetry thermogram shown in FIG. 7.

The 2:1 co-crystal formed by D-α-tocopherol and L-proline is characterized by the infrared absorption spectrum obtained by using a Fourier transform infrared spectrometer, and is characterized by the following important wave numbers: 3436 cm⁻¹, 3164 cm⁻¹, 2926 cm⁻¹, 1625 cm⁻¹, 1462cm⁻¹, 1377 cm⁻¹, 1316 cm⁻¹, 1292 cm⁻¹, 1261 cm⁻¹, 1225 cm⁻¹, 1171 cm⁻¹, 1089 cm⁻¹, 1033 cm⁻¹, 924 cm⁻¹, 865 cm⁻¹, 812 cm⁻¹, 787 cm⁻¹, 731 cm⁻¹, 484 cm⁻¹; preferably, it has an infrared spectrum shown in FIG. 8.

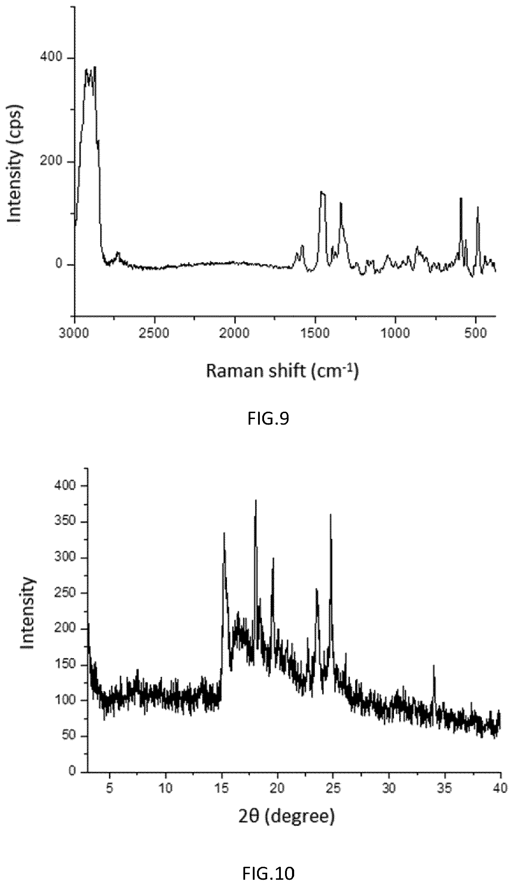

The 2:1 co-crystal formed by D-α-tocopherol and L-proline is characterized by the Raman spectrum obtained by using a laser Raman spectrometer, and is characterized by the following important wave numbers: 2921 cm⁻¹, 2896 cm⁻¹, 2870 cm⁻¹, 1615 cm⁻¹, 1582 cm⁻¹, 1462 cm⁻¹, 1393 cm⁻¹, 1340 cm⁻¹, 595 cm⁻¹, 564 cm⁻¹, 489 cm⁻¹; preferably, it has a Raman spectrum shown in FIG. 9.

The present invention also provides another co-crystal of D-α-tocopherol and L-proline. The co-crystal is in a partial crystalline form under normal pressure at room temperature. The stoichiometric ratio of D-α-tocopherol to L-proline in the co-crystal is 1:2. It is characterized by an X-ray powder diffraction pattern having characteristic peaks at 2θ diffraction angle of 15.2±0.2, 18.0±0.2, 19.6±0.2, 23.5±0.2, 24.8±0.2 degrees; preferably, the X-ray powder diffraction pattern further has characteristic peaks at 2θ diffraction angle of 7.5±0.2, 13.2±0.2, 18.4±0.2, 22.7±0.2 degrees; more preferably, it has an X-ray powder diffraction pattern shown in FIG. 10.

The preparation method for the above co-crystal is as follows: D-α-tocopherol and L-proline are added at a stoichiometric ratio of 1:2 to methanol solvent, and the resultant is stirred at 20-60°C for 0.5-2 hours, and recrystallized to obtain a white precipitate. The precipitate is filtered and the solid thus obtained is dried at room temperature to give a co-crystal of D-α-tocopherol and L-proline.

Preferably, the preparation method for the above co-crystal is as follows: D-α-tocopherol (5 mmol) and L-proline (10 mmol) are added at a stoichiometric ratio of 1:2 to 10 ml of methanol solvent, and the resultant is stirred at 40°C for 1 hour, and recrystallized to obtain a white precipitate. The precipitate is filtered through a Buchner funnel, and the solid is dried in a vacuum oven at room temperature for 1 day to give a co-crystal of D-α-tocopherol and L-proline.

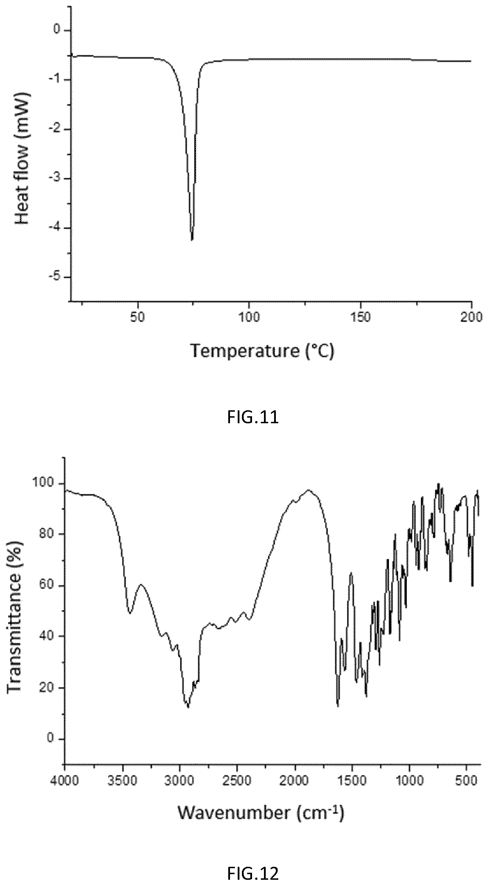

The 1:2 co-crystal formed by D-α-tocopherol and L-proline is determined by differential scanning calorimetry, the melting initiation temperature is 70±5°C, and the maximum peak value is 74°C; preferably, it has a differential scanning calorimetry thermogram shown in FIG. 11.

The 1:2 co-crystal formed by D-α-tocopherol and L-proline is characterized by the infrared absorption spectrum obtained by using a Fourier transform infrared spectrometer, and is characterized by the following important wave numbers: 3434 cm⁻¹, 2926 cm⁻¹, 1625 cm⁻¹, 1564 cm⁻¹, 1461 cm⁻¹, 1377 cm⁻¹, 1317 cm⁻¹, 1293 cm⁻¹, 1261 cm⁻¹, 1170 cm⁻¹, 1088 cm⁻¹, 1034 cm⁻¹, 944 cm⁻¹, 920 cm⁻¹, 850 cm⁻¹, 788 cm⁻¹, 670 cm⁻¹, 643 cm⁻¹, 484 cm⁻¹; preferably, it has an infrared spectrum shown in FIG. 12.

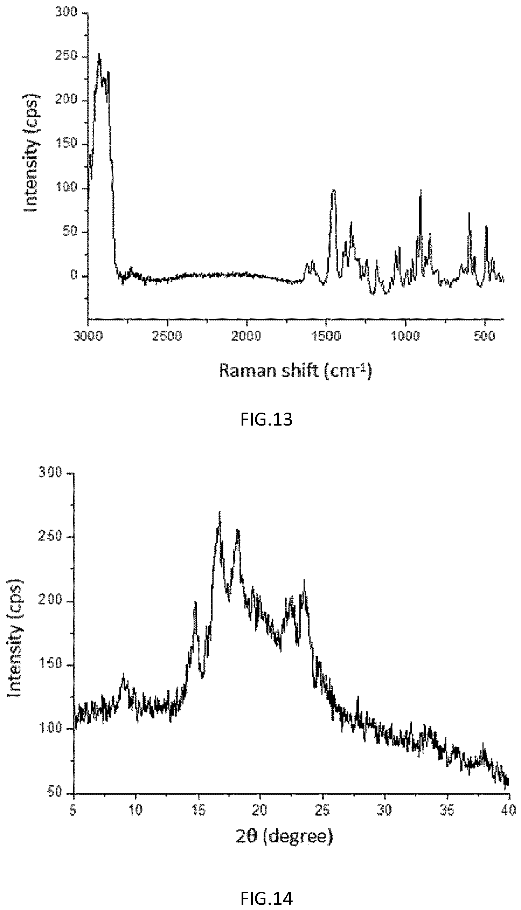

The 1:2 co-crystal formed by D-α-tocopherol and L-proline is characterized by the Raman spectrum obtained by using a laser Raman spectrometer, and is characterized by the following important wave numbers: 2971 cm⁻¹, 2872 cm⁻¹, 1619 cm⁻¹, 1582 cm⁻¹, 1449 cm⁻¹, 1377 cm⁻¹, 1341 cm⁻¹, 1243 cm⁻¹, 1180 cm⁻¹, 1061 cm⁻¹, 1038 cm⁻¹, 990 cm⁻¹, 956 cm⁻¹, 595 cm⁻¹, 924 cm⁻¹, 904 cm⁻¹, 867 cm⁻¹, 596 cm⁻¹, 489 cm⁻¹; preferably, it has a Raman spectrum shown in FIG. 13.

In another preferred embodiment of the present invention, the tocopherol co-crystal is a co-crystal of D-δ-tocopherol and D-proline.

Provided is a co-crystal of D-δ-tocopherol and D-proline. The co-crystal is in a partial crystalline form under normal pressure at room temperature. The stoichiometric ratio of D-δ-tocopherol to D-proline in the co-crystal is 3:2. It is characterized by an X-ray powder diffraction pattern having characteristic peaks at 2θ diffraction angle of 9.0±0.2, 14.8±0.2, 16.7±0.2, 18.2±0.2, 23.5±0.2 degrees; preferably, it has an X-ray powder diffraction pattern shown in FIG. 14.

The preparation method for the above co-crystal is as follows: D-δ-tocopherol and D-proline are added at a stoichiometric ratio of 3:2 to methanol, and the resultant is stirred at 20-60°C for 0.5-2 hours, and recrystallized to obtain a white precipitate. The precipitate is filtered and the solid thus obtained is dried at room temperature to give a co-crystal of D-δ-tocopherol and D-proline.

Preferably, the preparation method for the above co-crystal is as follows: D-δ-tocopherol (6 mmol) and D-proline (4 mmol) are added at a stoichiometric ratio of 3:2 to 10 ml of methanol, and the resultant is stirred at 40°C for 1 hour, and recrystallized to obtain a white precipitate. The precipitate is filtered through a Buchner funnel, and the solid is dried in a vacuum oven at room temperature for 1 day to give a co-crystal of D-δ-tocopherol and D-proline.

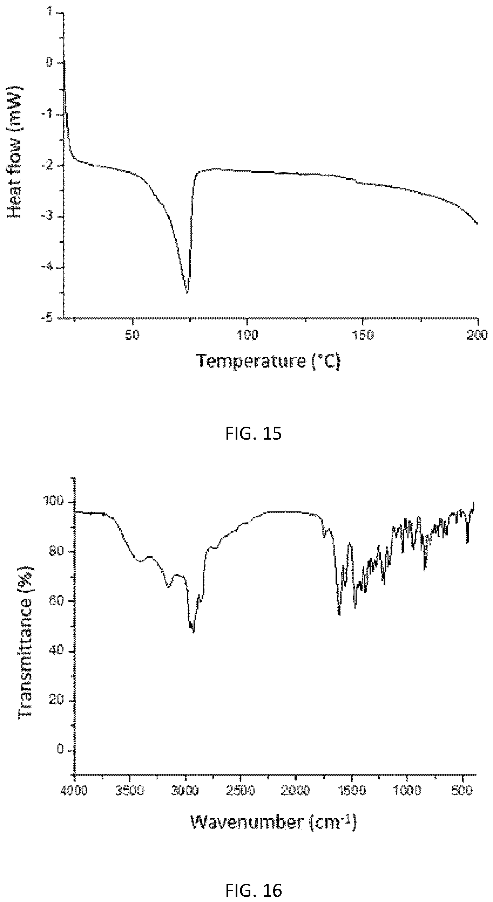

The 3:2 co-crystal formed by the D-δ-tocopherol and D-proline is determined by differential scanning calorimetry, the melting initiation temperature is 64±3°C, and the maximum peak value is 74°C; preferably, it has a differential scanning calorimetry thermogram shown in FIG. 15.

The 3:2 co-crystal formed by the D-δ-tocopherol and D-proline is characterized by the infrared absorption spectrum obtained by using a Fourier transform infrared spectrometer, and is characterized by the following important wave numbers: 3410 cm⁻¹, 3151 cm⁻¹, 2952 cm⁻¹, 2926 cm⁻¹, 1612 cm⁻¹, 1558 cm⁻¹, 1469 cm⁻¹, 1376 cm⁻¹, 1218 cm⁻¹, 1202 cm⁻¹, 1097 cm⁻¹, 1039 cm⁻¹, 993 cm⁻¹, 946 cm⁻¹, 874 cm⁻¹, 843 cm⁻¹, 674 cm⁻¹, 642 cm⁻¹; preferably, it has an infrared spectrum shown in FIG. 16.

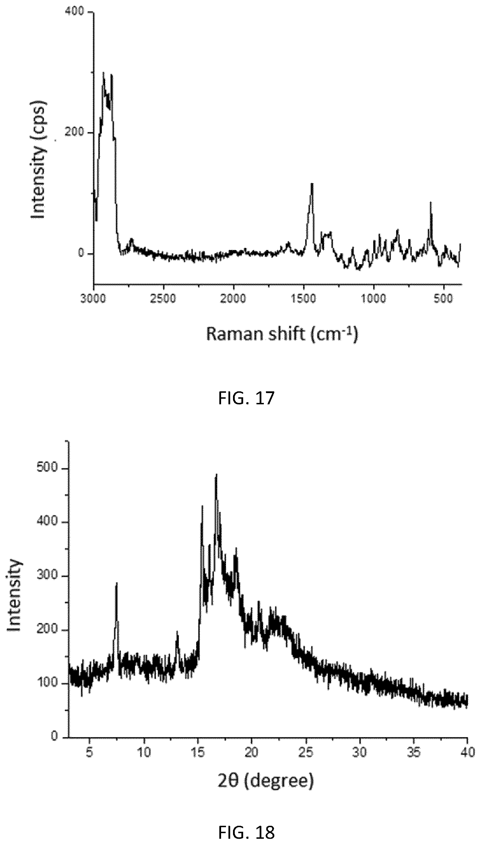

The 3:2 co-crystal formed by the D-δ-tocopherol and D-proline is characterized by the Raman spectrum obtained by using a laser Raman spectrometer, and is characterized by the following important wave numbers: 2999 cm⁻¹, 2930 cm⁻¹, 2873 cm⁻¹, 1443 cm⁻¹, 1376 cm⁻¹, 1152 cm⁻¹, 1052 cm⁻¹, 997 cm⁻¹, 962 cm⁻¹, 918 cm⁻¹, 748cm⁻¹, 595 cm⁻¹; preferably, it has a Raman spectrum shown in FIG. 17.

In another preferred embodiment of the present invention, the tocopherol co-crystal is a co-crystal of synthetic tocopherol and L-proline.

Provided is a co-crystal of synthetic tocopherol and L-proline. The co-crystal is in a partial crystalline form under normal pressure at room temperature. The stoichiometric ratio of synthetic tocopherol to L-proline in the co-crystal is 1:1. It is characterized by an X-ray powder diffraction pattern having characteristic peaks at 2θ diffraction angle of 7.5±0.2, 13.1±0.2, 15.4±0.2, 16.7±0.2, 18.6±0.2 degrees; preferably, the X-ray powder diffraction pattern further has characteristic peaks at 2θ diffraction angle of 16.1±0.2, 17.0±0.2 degrees; more preferably, it has an X-ray powder diffraction pattern shown in FIG. 18.

The preparation method for the above co-crystal is as follows: synthetic tocopherol and L-proline are added at a stoichiometric ratio of 1:1 to methanol, and the resultant is stirred at 20-40°C for 0.5-2 hours, and recrystallized to obtain a white precipitate. The precipitate is filtered and the solid thus obtained is dried at room temperature to give a co-crystal of synthetic tocopherol and L-proline.

Preferably, the preparation method for the above co-crystal is as follows: synthetic tocopherol (5 mmol) and L-proline (5 mmol) are added at a stoichiometric ratio of 1:1 to 10 ml of methanol, and the resultant is stirred at 40°C for 1 hour, and recrystallized to obtain a white precipitate. The precipitate is filtered through a Buchner funnel, and the solid is dried in a vacuum oven at room temperature for 1 day to give a co-crystal of synthetic tocopherol and L-proline.

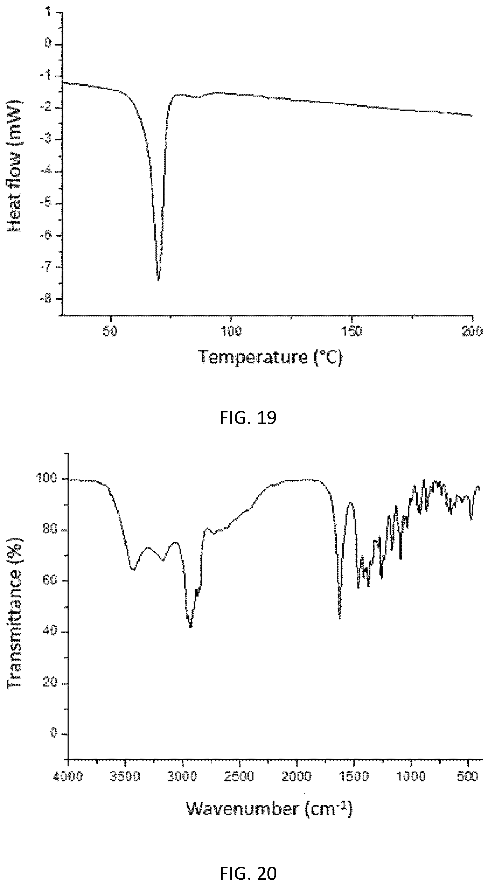

The 1:1 co-crystal formed by synthetic tocopherol and L-proline is determined by differential scanning calorimetry, the melting initiation temperature is 65±3°C, and the maximum peak value is 70°C; preferably, it has a differential scanning calorimetry thermogram shown in FIG. 19.

The 1:1 co-crystal formed by synthetic tocopherol and L-proline is characterized by the infrared absorption spectrum obtained by using a Fourier transform infrared spectrometer, and is characterized by the following important wave numbers: 3428 cm⁻¹, 3171 cm⁻¹, 2927 cm⁻¹, 1625 cm⁻¹, 1463 cm⁻¹, 1376 cm⁻¹, 1262 cm⁻¹, 1170 cm⁻¹, 1091 cm⁻¹, 1032 cm⁻¹, 927 cm⁻¹, 867 cm⁻¹, 670 cm⁻¹, 480 cm⁻¹; preferably, it has an infrared spectrum shown in FIG. 20.

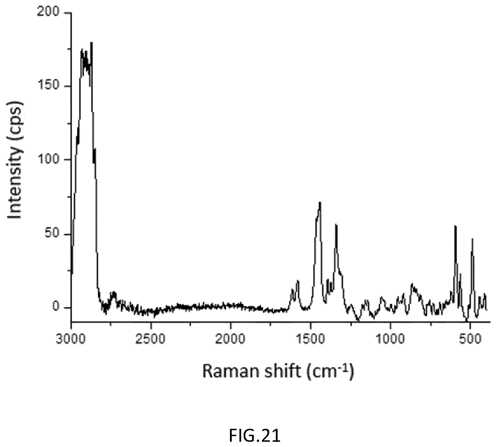

The 1:1 co-crystal formed by synthetic tocopherol and L-proline is characterized by the Raman spectrum obtained by using a laser Raman spectrometer, and is characterized by the following important wave numbers: 1614 cm⁻¹, 1580 cm⁻¹, 1442 cm⁻¹, 1394 cm⁻¹, 1374 cm⁻¹, 1340 cm⁻¹, 623 cm⁻¹, 596 cm⁻¹, 565 cm⁻¹, 489 cm⁻¹; preferably, it has a Raman spectrum shown in FIG. 21.

The invention also provides a health product, food, cosmetics, medicine, pharmaceutic adjuvant or feed, which contains the co-crystal of tocopherol and proline.

The invention also provides a use of the co-crystal of tocopherol and proline in the preparation of vitamin nutrition, antioxidants and the like.

The invention also provides a use of the co-crystal of tocopherol and proline in the preparation of health product, food, cosmetics, medicine, pharmaceutic adjuvant and feed.

The X-ray powder diffraction patterns in the present invention were obtained by using an X-ray powder diffractometer (Bruker D8 Advanced), with Cu Kα radiation(λ=1.54056Å) in a scanning range of 3° to 40° in 2θ at a scanning rate of 2°/min.

The differential scanning calorimetry was performed by using TA DSC Q2000 instrument at a heating rate of 10 K/min.

The Fourier transform infrared spectrometer used was Thermo Scientific Nicolet 6700.

The laser Raman spectrometer used was Thermo Scientific DXR Raman Microscope.

The co-crystals of the present invention are completely different from the mixture of tocopherol and proline obtained by simple physical mixing of the two compounds, and the present invention has the following beneficial effects:
1. Compared with the complex of tocopherol, the present co-crystals have better crystallinity and higher melting points; 2. Compared with the tocopherol itself, the present co-crystals have significantly improved stability; 3. Compared with semi-synthetic or fully synthetic tocopherol, the present co-crystals have more excellent antioxidant effect. Therefore, the present co- crystals of tocopherol and proline have high application value in practice.

### Brief Description of the Figures

FIG. 1 is an X-ray powder diffraction (XRPD) pattern of the crystalline form of the 1:1 co-crystal formed by D-α-tocopherol and L-proline provided by the present invention;
FIG. 2 is an X-ray powder diffraction (XRPD) pattern of the partial crystalline form of the 1:1 co-crystal formed by D-α-tocopherol and L-proline provided by the present invention;
FIG. 3 is a differential scanning calorimetry (DSC) thermogram of the crystalline form/partial crystalline form of the 1:1 co-crystal formed by D-α-tocopherol and L-proline provided by the present invention;
FIG. 4 is an infrared spectrum (IR) of the crystalline form/partial crystalline form of the 1:1 co-crystal formed by D-α-tocopherol and L-proline provided by the present invention;
FIG. 5 is a Raman spectrum (Raman) of the crystalline form/partial crystalline of the 1:1 co-crystal form formed by D-α-tocopherol and L-proline provided by the present invention;
FIG. 6 is an X-ray powder diffraction (XRPD) pattern of the partial crystalline form of the 2:1 co-crystal formed by D-α-tocopherol and L-proline provided by the present invention;
FIG. 7 is a differential scanning calorimetry (DSC) thermogram of the partial crystalline form of the 2:1 co-crystal formed by D-α-tocopherol and L-proline provided by the present invention;
FIG. 8 is an infrared spectrum (IR) of the partial crystalline form of the 2:1 co-crystal formed by D-α-tocopherol and L-proline provided by the present invention;
FIG. 9 is a Raman spectrum (Raman) of the partial crystalline form of the 2:1 co-crystal formed by D-α-tocopherol and L-proline provided by the present invention;
FIG. 10 is an X-ray powder diffraction (XRPD) pattern of the partial crystalline form of the 1:2 co-crystal formed by D-α-tocopherol and L-proline provided by the present invention;
FIG. 11 is a differential scanning calorimetry (DSC) thermogram of the partial crystalline form of the 1:2 co-crystal formed by D-α-tocopherol and L-proline provided by the present invention;
FIG. 12 is an infrared spectrum (IR) of the partial crystalline form of the 1:2 co-crystal formed by D-α-tocopherol and L-proline provided by the present invention;
FIG. 13 is a Raman spectrum (Raman) of the partial crystalline form of the 1:2 co-crystal formed by D-α-tocopherol and L-proline provided by the present invention;
FIG. 14 is an X-ray powder diffraction (XRPD) pattern of the partial crystalline form of the 3:2 co-crystal formed by D-δ-tocopherol and D-proline provided by the present invention;
FIG. 15 is a differential scanning calorimetry (DSC) thermogram of the partial crystalline form of the 3:2 co-crystal formed by D-δ-tocopherol and D-proline provided by the present invention;
FIG. 16 is an infrared spectrum (IR) of the partial crystalline form of the 3:2 co-crystal formed by D-δ-tocopherol and D-proline provided by the present invention;
FIG. 17 is a Raman spectrum (Raman) of the partial crystalline form of the 3:2 co-crystal formed by D-δ-tocopherol and D-proline provided by the present invention;
FIG. 18 is an X-ray powder diffraction (XRPD) pattern of the partial crystalline form of the 1:1 co-crystal formed by synthetic tocopherol and L-proline provided by the present invention;
FIG. 19 is a differential scanning calorimetry (DSC) thermogram of the partial crystalline form of the 1:1 co-crystal formed by synthetic tocopherol and L-proline provided by the present invention;
FIG. 20 is an infrared spectrum (IR) of the partial crystalline form of the 1:1 co-crystal formed by synthetic tocopherol and L-proline provided by the present invention;
FIG. 21 is a Raman spectrum (Raman) of the partial crystalline form of the 1:1 co-crystal formed by synthetic tocopherol and L-proline provided by the present invention;
FIG. 22 shows the comparison between the DPPH solution free radical scavenging ability of the synthetic tocopherol acetate and that of the 1:1 co-crystal formed by D-α-tocopherol and L-proline prepared in Example 2;
FIG. 23 shows a comparative picture of the antioxidant effects in the raw beef between the synthetic tocopherol acetate and the 1:1 co-crystal formed by D-α-tocopherol and L-proline prepared in Example 2.

### Detailed embodiments

In order to make the objectives, technical solutions and advantages of the present invention clearer, the present invention will be further described in detail below with reference to the drawings and examples. It should be understood that the embodiments described herein are only used to explain the present invention, and are not intended to limit the present invention.

### Example 1

### The co-crystal formed by D-α-tocopherol and L-proline at a stoichiometric ratio of 1:1

D-α-tocopherol (5 mmol) and L-proline (5 mmol) were added at a stoichiometric ratio of 1:1 to 20 ml of a mixed solvent of methanol and nitromethane (volume ratio 1: 1), and the resultant was stirred at 40°C for 1 hour, and recrystallized to obtain a white precipitate. The precipitate was filtered through a Buchner funnel, and the solid was dried in a vacuum oven at room temperature for 1 day to give a co-crystal of D-α-tocopherol and L-proline.

This co-crystal is in a crystalline form under normal pressure at room temperature, and the co-crystal was characterized by X-ray powder diffraction (XRPD). The result is shown in FIG. 1.

### Example 2

### The co-crystal formed by D-α-tocopherol and L-proline at a stoichiometric ratio of 1:1

D-α-tocopherol (5 mmol) and L-proline (5 mmol) were added at a stoichiometric ratio of 1:1 to 10 ml of methanol solvent, and the resultant was stirred at 40°C for 1 hour, and recrystallized to obtain a white precipitate. The precipitate was filtered through a Buchner funnel, and the solid was dried in a vacuum oven at room temperature for 1 day to give a co-crystal of D-α-tocopherol and L-proline.

Various qualitative methods can be used to confirm the formation of the co-crystal of D-α-tocopherol and L-proline in Example 2. This co-crystal is in a partial crystalline form under normal pressure at room temperature. The co-crystal was characterized by solid-state methods such as X-ray powder diffraction (XRPD), differential scanning calorimetry (DSC), infrared spectroscopy (IR), and Raman spectroscopy (Raman). The results are shown in FIGS. 2-5, respectively.

### Example 3

### The co-crystal formed by D-α-tocopherol and L-proline at a stoichiometric ratio of 2:1

D-α-tocopherol (10 mmol) and L-proline (5 mmol) were added at a stoichiometric ratio of 2:1 to 10 ml of methanol solvent, and the resultant was stirred at 40°C for 1 hour, and recrystallized to obtain a white precipitate. The precipitate was filtered through a Buchner funnel, and the solid was dried in a vacuum oven at room temperature for 1 day to give a co-crystal of D-α-tocopherol and L-proline.

Various qualitative methods can be used to confirm the formation of the co-crystal of D-α-tocopherol and L-proline in Example 3. This co-crystal is in a partial crystalline form under normal pressure at room temperature. The co-crystal was characterized by solid-state methods such as X-ray powder diffraction (XRPD), differential scanning calorimetry (DSC), infrared spectroscopy (IR), and Raman spectroscopy (Raman). The results are shown in FIGS. 6-9, respectively.

### Example 4

### The co-crystal formed by D-α-tocopherol and L-proline at a stoichiometric ratio of 1:2

D-α-tocopherol (5 mmol) and L-proline (10 mmol) were added at a stoichiometric ratio of 1:2 to 10 ml of methanol solvent, and the resultant was stirred at 40°C for 1 hour, and recrystallized to obtain a white precipitate. The precipitate was filtered through a Buchner funnel, and the solid was dried in a vacuum oven at room temperature for 1 day to give a co-crystal of D-α-tocopherol and L-proline.

Various qualitative methods can be used to confirm the formation of the co-crystal of D-α-tocopherol and L-proline in Example 4. This co-crystal is in a partial crystalline form under normal pressure at room temperature. The co-crystal was characterized by solid-state methods such as X-ray powder diffraction (XRPD), differential scanning calorimetry (DSC), infrared spectroscopy (IR), and Raman spectroscopy (Raman). The results are shown in FIGS. 10-13, respectively.

### Example 5

### The co-crystal formed by D-δ-tocopherol and D-proline at a stoichiometric ratio of 3:2

D-δ-tocopherol (6 mmol) and D-proline (4 mmol) were added at a stoichiometric ratio of 1:1 to 10 ml of methanol solvent, and the resultant was stirred at 40°C for 1 hour, and recrystallized to obtain a white precipitate. The precipitate was filtered through a Buchner funnel, and the solid was dried in a vacuum oven at room temperature for 1 day to give a co-crystal of D-δ-tocopherol and D-proline.

Various qualitative methods can be used to confirm the formation of the co-crystal of D-δ-tocopherol and D-proline in Example 5. This co-crystal is in a partial crystalline form under normal pressure at room temperature. The co-crystal was characterized by solid-state methods such as X-ray powder diffraction (XRPD), differential scanning calorimetry (DSC), infrared spectroscopy (IR), and Raman spectroscopy (Raman). The results are shown in FIGS. 14-17, respectively.

### Example 6

### The co-crystal formed by synthetic tocopherol and L-proline at a stoichiometric ratio of 1:1

Synthetic tocopherol (5 mmol) and L-proline (5 mmol) were added at a stoichiometric ratio of 1:1 to 10 ml of methanol solvent, and the resultant was stirred at 40°C for 1 hour, and recrystallized to obtain a white precipitate. The precipitate was filtered through a Buchner funnel, and the solid was dried in a vacuum oven at room temperature for 1 day to give a co-crystal of synthetic tocopherol and L-proline.

Various qualitative methods can be used to confirm the formation of the co-crystal of synthetic tocopherol and L-proline in Example 6. This co-crystal is in a partial crystalline form under normal pressure at room temperature. The co-crystal was characterized by solid-state methods such as X-ray powder diffraction (XRPD), differential scanning calorimetry (DSC), infrared spectroscopy (IR), and Raman spectroscopy (Raman). The results are shown in FIGS. 18-21, respectively.

### Example 7 (Comparison between the stability of D-α-tocopherol itself and that of the co-crystal of D-α-tocopherol and L-proline)

Those skilled in the art can understand that the co-crystals of the present invention in the context have significantly improved stability compared to D-α-tocopherol itself; representative studies were carried out by using the 1:1 co-crystal formed by D-α-tocopherol and L-proline obtained in Example 2.

The co-crystal of D-α-tocopherol and L-proline obtained in Example 2 was compared with D-α-tocopherol itself for their difference in stability, including (1) high temperature stability at 60°C, (2) 5500 lux light stability, (3) accelerated test stability (40°C/75% relative humidity), (4) long-term test stability (25°C/60% relative humidity).

The results are shown in Table 1 to Table 8.
Table 1 shows the content change of D-α-tocopherol under high temperature conditions;
Table 2 shows the content change of the 1:1 co-crystal formed by D-α-tocopherol and L-proline under high temperature conditions;
Table 3 shows the content change of D-α-tocopherol under the condition of strong light irradiation;
Table 4 shows the content change of the 1:1 co-crystal formed by D-α-tocopherol and L-proline under the condition of strong light irradiation;
Table 5 shows the content change of D-α-tocopherol under accelerated test conditions (40°C/75% relative humidity);
Table 6 shows the content change of the 1:1 co-crystal formed by D-α-tocopherol and L-proline under accelerated test conditions (40°C/75% relative humidity);
Table 7 shows the content change of D-α-tocopherol under long-term test conditions (25°C /60% relative humidity);
Table 8 shows the content change of the 1:1 co-crystal formed by D-α-tocopherol and L-proline under long-term test conditions (25°C/60% relative humidity).

**Table 1**

| **Item** | **High temperature test (60 °C)** | | | | |
|---|---|---|---|---|---|
| | **Day 0** | **Day 5** | **Day 10** | **Day 20** | **Day 30** |
| Appearance | Light yellow oily liquid | Light yellow oily liquid | Yellow oily liquid | Yellow oily liquid | Yellow oily liquid |
| content | 100% | 96.7% | 89.4% | 78.7% | 77.3% |

**Table 2**

| **Item** | **High temperature test (60 °C)** | | | | |
|---|---|---|---|---|---|
| | **Day 0** | **Day 5** | **Day 10** | **Day 20** | **Day 30** |
| Appearance | Off-white powder | Off-white powder | Off-white powder | Off-white powder | Off-white powder |
| content | 100% | 98.5% | 96.9% | 96.9% | 95.4% |

**Table 3**

| **Item** | **Strong light irradiation test (25 °C, illuminance 5500 Lux, near ultraviolet energy 90µW/cm²)** | | | |
|---|---|---|---|---|
| | **Day 0** | **Day 5** | **Day 10** | **Day 20** |
| Appearance | Light yellow oily liquid | Light yellow oily liquid | Light yellow oily liquid | Light yellow oily liquid |
| content | 100% | 97.8% | 91.8% | 88.5% |

**Table 4**

| **Item** | **Strong light irradiation test (25 °C, illuminance 5500 Lux, near ultraviolet energy 90µW/cm²)** | | | |
|---|---|---|---|---|
| | **Day 0** | **Day 5** | **Day 10** | **Day 20** |
| Appearance | Off-white powder | Off-white powder | Off-white powder | Off-white powder |
| content | 100% | 96.3% | 96.1% | 97.3% |

**Table 5**

| **Item** | **Time (Month)** | | | |
|---|---|---|---|---|
| | **0** | **1** | **2** | **3** |
| Appearance | Yellow liquid | Yellow liquid | Yellow liquid | Yellow liquid |
| content | 100% | 98.0% | 99.1% | 96.9% |

**Table 6**

| **Item** | **Time (Month)** | | | |
|---|---|---|---|---|
| | **0** | **1** | **2** | **3** |
| Appearance | Off-white powder | Off-white powder | Off-white powder | Off-white powder |
| content | 100% | 100.6% | 100.1% | 99.7% |

**Table 7**

| **Item** | **Time (Month)** | |
|---|---|---|
| | **0** | **3** |
| Appearance | Yellow liquid | Yellow liquid |
| content | 100.0% | 96.9% |

**Table 8**

| **Item** | **Time (Month)** | |
|---|---|---|
| | **0** | **3** |
| Appearance | Off-white powder | Off-white powder |
| content | 100.0% | 99.6% |

The content was determined by HPLC, and the content was calculated by the external standard method. The initial contents of D-α-tocopherol in D-α-tocopherol itself and the co-crystal were both set at 100%. At 60°C, after 30 days, the content of D-α-tocopherol itself was 77.3% of the initial content, while the content of D-α-tocopherol in the 1:1 co-crystal formed by D-α-tocopherol and L-proline was maintained at 95.4%; under the conditions of strong light irradiation, after 20 days, the content of D-α-tocopherol itself was 88.5% of the initial content, while the content of D-α-tocopherol in the 1:1 co-crystal formed by D-α-tocopherol and L-proline was maintained at 97.3%; under accelerated test conditions (40°C/75% relative humidity), after 3 months, the content of D-α-tocopherol itself was 96.9% of the initial content, while the content of D-α-tocopherol in the 1:1 co-crystal formed by D-α-tocopherol and L-proline was maintained at 99.7%; under long-term test conditions (25°C /60% Relative humidity), after 3 months, the content of D-α-tocopherol itself was 96.9% of the initial content, while the content of D-α-tocopherol in the 1:1 co-crystal formed by D-α-tocopherol and L-proline was maintained at 99.6%. It can be seen that the co-crystal of D-α-tocopherol and L-proline has better high-temperature stability, light stability, accelerated stability and long-term stability when compared with D-α-tocopherol raw material itself.

### Example 8 (Stability Comparison between synthetic tocopherol itself and the co-crystal of synthetic tocopherol and L-proline)

Those skilled in the art can understand that the co-crystal of the present invention in the context has a significantly improved stability compared to the synthetic tocopherol itself; representative studies were carried out by using the 1:1 co-crystal formed by synthetic tocopherol and L-proline obtained in Example 6.

The acceleration test (40°C/75% relative humidity) was carried out for the comparison of the stability difference between the co-crystal of synthetic tocopherol and L-proline obtained in Example 6 and synthetic tocopherol itself. The results are shown in Table 9-Table 10. The content was determined by HPLC, the content was calculated by the external standard method, and the content is calculated based on the total tocopherol content. Both the initial tocopherol contents in the synthetic tocopherol itself and in the co-crystals were set at 100%. Under the accelerated test conditions, after 1 month, the content of synthetic tocopherol itself was 94.1% of the initial content, while the content of tocopherol in the 1:1 co-crystal formed by synthetic tocopherol and L-proline remained unchanged. It can be seen that the co-crystal of synthetic tocopherol and L-proline has better acceleration stability when compared with the synthetic tocopherol raw material itself.

Table 9 shows the content change of synthetic tocopherol under accelerated test conditions (40°C/75% relative humidity);
Table 10 shows the content change of the 1:1 co-crystal formed by synthetic tocopherol and L-proline under accelerated test conditions (40°C/75% relative humidity).

**Table 9**

| **Item** | **Time (Month)** | |
|---|---|---|
| | **0** | **1** |
| Appearance | Yellow liquid | Yellow liquid |
| content | 100.0% | 94.1% |

**Table 10**

| **Item** | **Time (Month)** | |
|---|---|---|
| | **0** | **1** |
| Appearance | Yellow liquid | Yellow liquid |
| content | 100.0% | 100.2% |

### Example 9 (Comparison between the free radical scavenging ability of synthetic tocopherol acetate and that of the co-crystal of D-α-tocopherol and L-proline)

Those skilled in the art can understand that the co-crystals of the present invention in the context have a stronger ability to scavenge free radicals when compared with synthetic tocopherol acetate.

Experimental reagents: 1:1 co-crystal formed by D-α-tocopherol and L-proline prepared in Example 2; commercially available synthetic tocopherol acetate (50% embedded powder of synthetic tocopherol acetate (active ingredients comprise the acetate derivatives of 8 kinds of α-tocopherol isomers, and the auxiliary materials are sodium starch octenyl succinate, maltodextrin, silica), purchased from DSM Inc.); methanol.

DPPH, also known as 1,1-diphenyl-2-trinitrophenylhydrazine, is a very stable nitrogen-centered free radical. Its solution with a purple color has maximum absorption at 517 nm and the absorbance is proportional to its concentration. The free radical scavenging ability of the compound is evaluated based on the principle that the color of solution becomes lighter after being added with a free radical scavenger, which reduces the number of free radicals, thereby decreasing the absorbance.

Sample preparation:
A. 5 mg/mL (D-α-tocopherol equivalent) of the solutions of the commercially available synthetic tocopherol acetate and the co-crystal of D-α-tocopherol and L-proline of the present invention in methanol were prepared.
B. 75 µM of the solution of DPPH in methanol was prepared;
C. As a blank control group, 0.8 mL of methanol was added to 9.2 mL of DPPH, and the resultant was kept in the dark for 30 min for UV absorption testing;
D. 0.8 mL of the solution of tocopherol was added to 9.2 mL of the solution of DPPH, and the resultant was kept in the dark for 30 min for UV absorption testing;

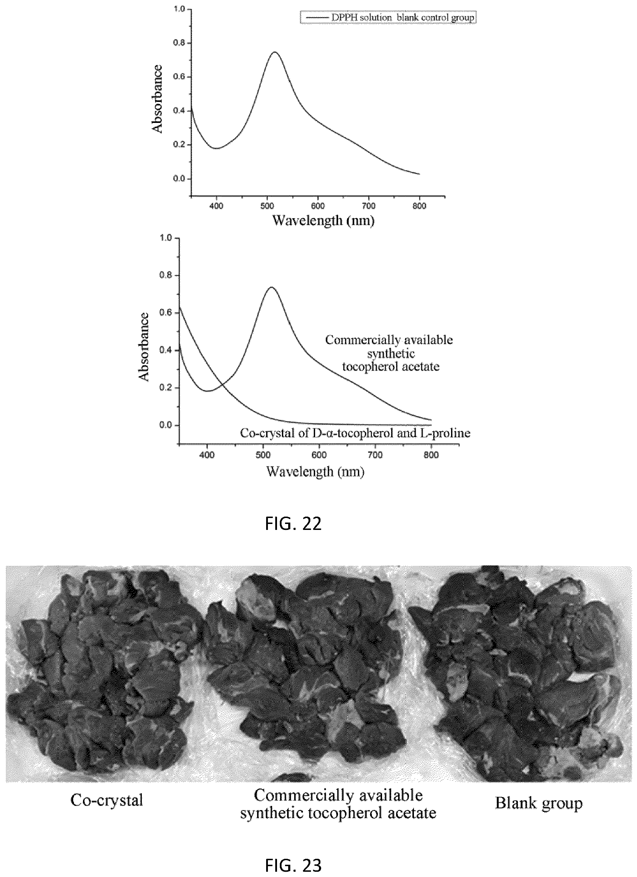

The results of ultraviolet absorption are shown in FIG. 22, it can be seen that, the co-crystal of the present invention can significantly reduce the absorption of DPPH solution at 517 nm wavelength, it proves that the co-crystal has prominent free radical scavenging ability, while the commercially available synthetic tocopherol acetate loses its free radical scavenging ability.

### Example 10 (Comparison between the antioxidant effect in raw beef of commercially available synthetic tocopherol acetate and that of the 1:1 co-crystal of D-α-tocopherol and L-proline)

Those skilled in the art can understand that, when compared with the commercially available synthetic tocopherol acetate, the co-crystal of the present invention in the context has a more significant antioxidant effect, which contributes to the stabilization of meat color, preservation and anti-oxidation of the meat.

Experimental reagents: 1:1 co-crystal formed by D-α-tocopherol and L-proline prepared in Example 2; commercially available synthetic tocopherol acetate (same as that in Example 8); 95% ethanol; commercially available bottled water.

Sample preparations:
A. the lean beef was cut into pieces with a size of about 2x2 cm, 200 grams for each.
B. 69mg of the co-crystal was dissolved in 5ml of 95% ethanol solution, and the same equivalent of the commercially available synthetic tocopherol acetate was dissolved in 5ml of commercially available bottled water. Each of the prepared tocopherol solution was put in a small spray bottle and sprayed evenly on the surface of the beef, into which the remaining ingredients were further added (co-crystal-treated sample was added with 5ml of commercially available bottled water; commercially available synthetic tocopherol acetate-treated sample was added with 5ml of 95% ethanol solution) and the treated beef was kneaded evenly for 2 minutes. As the blank control group, 200 g of beef was treated with 5ml of 95% ethanol solution and 5ml of commercially available bottled water.
C. All samples were placed in a metal dish, numbered and stored at 2-4°C for 8 days. Beef samples treated with different tocopherols were observed at different storage times for their variations in color and sensory indexes.

The results are shown in FIG. 23. After 8 days, there are significant differences in meat color among the sample added with the co-crystal of D-α-tocopherol and L-proline, the sample added with commercially available synthetic tocopherol acetate and the blank sample. The co-crystal of D-α-tocopherol and L-proline can delay the darkening and greening of beef; in terms of flavor, except for the sample added with the co-crystal of D-α-tocopherol and L-proline, both of the other two samples have rancidity. It can be seen that the addition of the co-crystal contributes to the stabilization of meat color, preservation and anti-oxidation of the meat.

The above contents are only the preferred embodiments of the present invention and are not intended to limit the present invention. Any modification, equivalence and improvement and the like made within the spirit and principle of the present invention shall be included in the protection scope of the present invention.

## Claims

1. A co-crystal of tocopherol and proline.

2. The co-crystal of tocopherol and proline of claim 1, **characterized in that**,
the proline comprises D-proline, or L-proline, or a racemic mixture of the both;
the tocopherol comprises natural tocopherol and synthetic tocopherol, the natural tocopherol comprises α-, β-, γ-, δ-tocopherols and α-, β-, γ-, δ-tocotrienols; the synthetic tocopherol comprises DL-α-tocopherols.

3. The co-crystal of tocopherol and proline of claim 1 or 2, **characterized in that**, the stoichiometric ratio of tocopherol to proline in the co-crystal is 1:2^{∼}2:1.

4. A method for preparing the co-crystal of tocopherol and proline of claim 1 or 2, **characterized in that**, the method comprises the following steps:
(i) Dissolving tocopherol and proline in a suitable solvent and stirring the same at 10-80°C until they are dissolved;
(ii) Recrystallizing the solution obtained from step (i);
(iii) Separating the solid and drying the same,
wherein, the suitable solvent is an organic solvent selected from water, methanol, ethanol, isopropanol, acetone, methyl ethyl ketone, acetonitrile, tetrahydrofuran, nitromethane, ethyl acetate, isopropyl acetate, methyl tert-butyl ether, toluene, DMF and methylene chloride and a mixed solvent thereof.

5. A co-crystal of tocopherol and proline, **characterized in that**, the co-crystal is a co-crystal of D-α-tocopherol and L-proline, the co-crystal is in a crystalline form under normal pressure at room temperature, the stoichiometric ratio of D-α-tocopherol to L-proline in the co-crystal is 1:1, the co-crystal is **characterized by** an X-ray powder diffraction pattern having characteristic peaks at 2θ diffraction angle of 3.8±0.2, 5.8±0.2, 7.7±0.2, 9.7±0.2, 11.6±0.2, 13.6±0.2, 15.4±0.2, 17.5±0.2, 21.4±0.2 degrees; preferably, the X-ray powder diffraction pattern further has characteristic peaks at 2θ diffraction angle of 16.8±0.2, 18.8±0.2, 19.5±0.2, 23.4±0.2 degrees; more preferably, the co-crystal has an X-ray powder diffraction pattern shown in FIG. 1.

6. The co-crystal of tocopherol and proline of claim 5, **characterized in that**, the co-crystal is determined by differential scanning calorimetry, the melting initiation temperature is 66±3°C, and the maximum peak value is 70°C; preferably, the co-crystal has a differential scanning calorimetry thermogram shown in FIG. 3.

7. The co-crystal of tocopherol and proline of claim 5, **characterized in that**, the co-crystal is **characterized by** the infrared absorption spectrum obtained by using a Fourier transform infrared spectrometer, and is **characterized by** the following important wave numbers: 3438 cm⁻¹, 3163 cm⁻¹, 2926 cm⁻¹, 1626 cm⁻¹, 1564 cm⁻¹, 1462 cm⁻¹, 1377 cm⁻¹, 1294 cm⁻¹, 1262 cm⁻¹, 1171 cm⁻¹, 1089 cm⁻¹, 1034 cm⁻¹, 923 cm⁻¹, 864 cm⁻¹, 670 cm⁻¹, 484 cm⁻¹; preferably, the co-crystal has an infrared spectrum shown in FIG. 4.

8. The co-crystal of tocopherol and proline of claim 5, **characterized in that**, the co-crystal is **characterized by** the Raman spectrum obtained by using a laser Raman spectrometer, and is **characterized by** the following important wave numbers: 2922 cm⁻¹, 2870 cm⁻¹, 1617 cm⁻¹, 1580 cm⁻¹, 1464 cm⁻¹, 1393 cm⁻¹, 1340 cm⁻¹, 596 cm⁻¹, 566 cm⁻¹, 491 cm⁻¹; preferably, the co-crystal has a Raman spectrum shown in FIG. 5.

9. A method for preparing the co-crystal of any one of claims 5 to 8, **characterized in that**, the method comprises the following steps: D-α-tocopherol and L-proline are added at a stoichiometric ratio of 1:1 to a mixed solvent of methanol and nitromethane, and the resultant is stirred at 20-60°C for 0.5-2 hours, and recrystallized to obtain a white precipitate, and the precipitate is filtered and the solid thus obtained is dried at room temperature to give a co-crystal of D-α-tocopherol and L-proline.

10. A co-crystal of tocopherol and proline, **characterized in that**, the co-crystal is a co-crystal of D-α-tocopherol and L-proline, the co-crystal is in a partial crystalline form under normal pressure at room temperature, and the stoichiometric ratio of D-α-tocopherol to L-proline in the co-crystal is 1:1; the co-crystal is **characterized by** an X-ray powder diffraction pattern having characteristic peaks at 2θ diffraction angle of 7.6±0.2, 13.2±0.2, 15.3±0.2, 18.1±0.2, 19.5±0.2, 23.5±0.2, 24.8±0.2 degrees; preferably, the co-crystal has an X-ray powder diffraction pattern shown in FIG. 2.

11. The co-crystal of tocopherol and proline of claim 10, **characterized in that**, the co-crystal is determined by differential scanning calorimetry, the melting initiation temperature is 66±3°C, and the maximum peak value is 70°C; preferably, the co-crystal has a differential scanning calorimetry thermogram shown in FIG. 3.

12. The co-crystal of tocopherol and proline of claim 10, **characterized in that**, the co-crystal is **characterized by** the infrared absorption spectrum obtained by using a Fourier transform infrared spectrometer, and is **characterized by** the following important wave numbers: 3438 cm⁻¹, 3163 cm⁻¹, 2926 cm⁻¹, 1626 cm⁻¹, 1564 cm⁻¹, 1462 cm⁻¹, 1377 cm⁻¹, 1294 cm⁻¹, 1262 cm⁻¹, 1171 cm⁻¹, 1089 cm⁻¹, 1034 cm⁻¹, 923 cm⁻¹, 864 cm⁻¹, 670 cm⁻¹, 484 cm⁻¹; preferably, the co-crystal has an infrared spectrum shown in FIG. 4.

13. The co-crystal of tocopherol and proline of claim 10, **characterized in that**, the co-crystal is **characterized by** the Raman spectrum obtained by using a laser Raman spectrometer, and is **characterized by** the following important wave numbers: 2922 cm⁻¹, 2870 cm⁻¹, 1617 cm⁻¹, 1580 cm⁻¹, 1464 cm⁻¹, 1393 cm⁻¹, 1340 cm⁻¹, 596 cm⁻¹, 566 cm⁻¹, 491 cm⁻¹; preferably, the co-crystal has a Raman spectrum shown in FIG. 5.

14. A method for preparing the co-crystal of any one of claims 10 to 13, **characterized in that**, the method comprises the following steps: D-α-tocopherol and L-proline are added at a stoichiometric ratio of 1:1 to methanol solvent, and the resultant is stirred at 20-60°C for 0.5-2 hours, and recrystallized to obtain a white precipitate, the precipitate is filtered and the solid thus obtained is dried at room temperature to give a co-crystal of D-α-tocopherol and L-proline.

15. A co-crystal of tocopherol and proline, **characterized in that**, the co-crystal is a co-crystal of D-α-tocopherol and L-proline, the co-crystal is in a partial crystalline form under normal pressure at room temperature; the stoichiometric ratio of D-α-tocopherol to L-proline in the co-crystal is 2:1; the co-crystal is **characterized by** an X-ray powder diffraction pattern having characteristic peaks at 2θ diffraction angle of 7.5±0.2, 13.2±0.2, 15.2±0.2, 23.4±0.2 degrees; preferably, the X-ray powder diffraction pattern further has characteristic peaks at 2θ diffraction angle of 9.4±0.2, 18.6±0.2, 19.9±0.2 degrees; more preferably, the co-crystal has an X-ray powder diffraction pattern shown in FIG. 6.

16. The co-crystal of tocopherol and proline of claim 15, **characterized in that**, the co-crystal is determined by differential scanning calorimetry, the melting initiation temperature is 73±5°C, and the maximum peak value is 77°C; preferably, the co-crystal has a differential scanning calorimetry thermogram shown in FIG. 7.

17. The co-crystal of tocopherol and proline of claim 15, **characterized in that**, the co-crystal is **characterized by** the infrared absorption spectrum obtained by using a Fourier transform infrared spectrometer, and is **characterized by** the following important wave numbers: 3436 cm⁻¹, 3164 cm⁻¹, 2926 cm⁻¹, 1625 cm⁻¹, 1462cm⁻¹, 1377 cm⁻¹, 1316 cm⁻¹, 1292 cm⁻¹, 1261 cm⁻¹, 1225 cm⁻¹, 1171 cm⁻¹, 1089 cm⁻¹, 1033 cm⁻¹, 924 cm⁻¹, 865 cm⁻¹, 812 cm⁻¹, 787 cm⁻¹, 731 cm⁻¹, 484 cm⁻¹; preferably, the co-crystal has an infrared spectrum shown in FIG. 8.

18. The co-crystal of tocopherol and proline of claim 15, **characterized in that**, the co-crystal is **characterized by** the Raman spectrum obtained by using a laser Raman spectrometer, and is **characterized by** the following important wave numbers: 2921 cm⁻¹, 2896 cm⁻¹, 2870 cm⁻¹, 1615 cm⁻¹, 1582 cm⁻¹, 1462 cm⁻¹, 1393 cm⁻¹, 1340 cm⁻¹, 595 cm⁻¹, 564 cm⁻¹, 489 cm⁻¹; preferably, the co-crystal has a Raman spectrum shown in FIG. 9.

19. A method for preparing the co-crystal of any one of claims 15 to 18, **characterized in that**, the method comprises the following steps: D-α-tocopherol and L-proline are added at a stoichiometric ratio of 2:1 to methanol solvent, and the resultant is stirred at 20-60°C for 0.5-2 hours, and recrystallized to obtain a white precipitate, the precipitate is filtered and the solid thus obtained is dried at room temperature to give a co-crystal of D-α-tocopherol and L-proline.

20. A co-crystal of tocopherol and proline, **characterized in that**, the co-crystal is a co-crystal of D-α-tocopherol and L-proline, and the co-crystal is in a partial crystalline form under normal pressure at room temperature; the stoichiometric ratio of D-α-tocopherol to L-proline in the co-crystal is 1:2; the co-crystal is **characterized by** an X-ray powder diffraction pattern having characteristic peaks at 2θ diffraction angle of 15.2±0.2, 18.0±0.2, 19.6±0.2, 23.5±0.2, 24.8±0.2 degrees; preferably, the X-ray powder diffraction pattern further has characteristic peaks at 2θ diffraction angle of 7.5±0.2, 13.2±0.2, 18.4±0.2, 22.7±0.2 degrees; more preferably, the co-crystal has an X-ray powder diffraction pattern shown in FIG. 10.

21. The co-crystal of tocopherol and proline of claim 20, **characterized in that**, the co-crystal is determined by differential scanning calorimetry, the melting initiation temperature is 70±5°C, and the maximum peak value is 74°C; preferably, the co-crystal has a differential scanning calorimetry thermogram shown in FIG. 11.

22. The co-crystal of tocopherol and proline of claim 20, **characterized in that**, the co-crystal is **characterized by** the infrared absorption spectrum obtained by using a Fourier transform infrared spectrometer, and is **characterized by** the following important wave numbers: 3434 cm⁻¹, 2926 cm⁻¹, 1625 cm⁻¹, 1564 cm⁻¹, 1461 cm⁻¹, 1377 cm⁻¹, 1317 cm⁻¹, 1293 cm⁻¹, 1261 cm⁻¹, 1170 cm⁻¹, 1088 cm⁻¹, 1034 cm⁻¹, 944 cm⁻¹, 920 cm⁻¹, 850 cm⁻¹, 788 cm⁻¹, 670 cm⁻¹, 643 cm⁻¹, 484 cm⁻¹; preferably, the co-crystal has an infrared spectrum shown in FIG. 12.

23. The co-crystal of tocopherol and proline of claim 20, **characterized in that**, the co-crystal is **characterized by** the Raman spectrum obtained by using a laser Raman spectrometer, and is **characterized by** the following important wave numbers: 2971 cm⁻¹, 2872 cm⁻¹, 1619 cm⁻¹, 1582 cm⁻¹, 1449 cm⁻¹, 1377 cm⁻¹, 1341 cm⁻¹, 1243 cm⁻¹, 1180 cm⁻¹, 1061 cm⁻¹, 1038 cm⁻¹, 990 cm⁻¹, 956 cm⁻¹, 595 cm⁻¹, 924 cm⁻¹, 904 cm⁻¹, 867 cm⁻¹, 596 cm⁻¹, 489 cm⁻¹; preferably, the co-crystal has a Raman spectrum shown in FIG. 13.

24. A method for preparing the co-crystal of any one of claims 20 to 23, **characterized in that**, the method comprises the following steps: D-α-tocopherol and L-proline are added at a stoichiometric ratio of 1:2 to methanol solvent, and the resultant is stirred at 20-60°C for 0.5-2 hours, and recrystallized to obtain a white precipitate, the precipitate is filtered and the solid thus obtained is dried at room temperature to give a co-crystal of D-α-tocopherol and L-proline.

25. A co-crystal of tocopherol and proline, **characterized in that**, the co-crystal is a co-crystal of D-δ-tocopherol and D-proline, and the co-crystal is in a partial crystalline form under normal pressure at room temperature; the stoichiometric ratio of D-δ-tocopherol to D-proline in the co-crystal is 3:2, the co-crystal is **characterized by** an X-ray powder diffraction pattern having characteristic peaks at 2θ diffraction angle of 9.0±0.2, 14.8±0.2, 16.7±0.2, 18.2±0.2, 23.5±0.2 degrees; preferably, the co-crystal has an X-ray powder diffraction pattern shown in FIG. 14.

26. The co-crystal of tocopherol and proline of claim 25, **characterized in that**, the co-crystal is determined by differential scanning calorimetry, the melting initiation temperature is 64±3°C, and the maximum peak value is 74°C; preferably, the co-crystal has a differential scanning calorimetry thermogram shown in FIG. 15.

27. The co-crystal of tocopherol and proline of claim 25, **characterized in that**, the co-crystal is **characterized by** the infrared absorption spectrum obtained by using a Fourier transform infrared spectrometer, and is **characterized by** the following important wave numbers: 3410 cm⁻¹, 3151 cm⁻¹, 2952 cm⁻¹, 2926 cm⁻¹, 1612 cm⁻¹, 1558 cm⁻¹, 1469 cm⁻¹, 1376 cm⁻¹, 1218 cm⁻¹, 1202 cm⁻¹, 1097 cm⁻¹, 1039 cm⁻¹, 993 cm⁻¹, 946 cm⁻¹, 874 cm⁻¹, 843 cm⁻¹, 674 cm⁻¹, 642 cm⁻¹; preferably, the co-crystal has an infrared spectrum shown in FIG. 16.

28. The co-crystal of tocopherol and proline of claim 25, **characterized in that**, the co-crystal is **characterized by** the Raman spectrum obtained by using a laser Raman spectrometer, and is **characterized by** the following important wave numbers: 2999 cm⁻¹, 2930 cm⁻¹, 2873 cm⁻¹, 1443 cm⁻¹, 1376 cm⁻¹, 1152 cm⁻¹, 1052 cm⁻¹, 997 cm⁻¹, 962 cm⁻¹, 918 cm⁻¹, 748cm⁻¹, 595 cm⁻¹; preferably, the co-crystal has a Raman spectrum shown in FIG. 17.

29. A method for preparing the co-crystal of any one of claims 25 to 28, **characterized in that**, the method comprises the following steps: D-δ-tocopherol and D-proline are added at a stoichiometric ratio of 3:2 to methanol, and the resultant is stirred at 20-60°C for 0.5-2 hours, and recrystallized to obtain a white precipitate, the precipitate is filtered and the solid thus obtained is dried at room temperature to give a co-crystal of D-δ-tocopherol and D-proline.

30. A co-crystal of tocopherol and proline, **characterized in that**, the co-crystal is a co-crystal of synthetic tocopherol and L-proline, and the co-crystal is in a partial crystalline form under normal pressure at room temperature; the stoichiometric ratio of synthetic tocopherol to L-proline in the co-crystal is 1:1; the co-crystal is **characterized by** an X-ray powder diffraction pattern having characteristic peaks at 2θ diffraction angle of 7.5±0.2, 13.1±0.2, 15.4±0.2, 16.7±0.2, 18.6±0.2 degrees; preferably, the X-ray powder diffraction pattern further has characteristic peaks at 2θ diffraction angle of 16.1±0.2, 17.0±0.2 degrees; more preferably, the co-crystal has an X-ray powder diffraction pattern shown in FIG. 18.

31. The co-crystal of tocopherol and proline of claim 30, **characterized in that**, the co-crystal is determined by differential scanning calorimetry, the melting initiation temperature is 65±3°C, and the maximum peak value is 70°C; preferably, the co-crystal has a differential scanning calorimetry thermogram shown in FIG. 19.

32. The co-crystal of tocopherol and proline of claim 30, **characterized in that**, the co-crystal is **characterized by** the infrared absorption spectrum obtained by using a Fourier transform infrared spectrometer, and is **characterized by** the following important wave numbers: 3428 cm⁻¹, 3171 cm⁻¹, 2927 cm⁻¹, 1625 cm⁻¹, 1463 cm⁻¹, 1376 cm⁻¹, 1262 cm⁻¹, 1170 cm⁻¹, 1091 cm⁻¹, 1032 cm⁻¹, 927 cm⁻¹, 867 cm⁻¹, 670 cm⁻¹, 480 cm⁻¹; preferably, the co-crystal has an infrared spectrum shown in FIG. 20.

33. The co-crystal of tocopherol and proline of claim 30, **characterized in that**, the co-crystal is **characterized by** the Raman spectrum obtained by using a laser Raman spectrometer, and is **characterized by** the following important wave numbers: 1614 cm⁻¹, 1580 cm⁻¹, 1442 cm⁻¹, 1394 cm⁻¹, 1374 cm⁻¹, 1340 cm⁻¹, 623 cm⁻¹, 596 cm⁻¹, 565 cm⁻¹, 489 cm⁻¹; preferably, the co-crystal has a Raman spectrum shown in FIG. 21.

34. A method for preparing the co-crystal of any one of claims 30 to 33, **characterized in that**, the method comprises the following steps: synthetic tocopherol and L-proline are added at a stoichiometric ratio of 1:1 to methanol, and the resultant is stirred at 20-40°C for 0.5-2 hours, and recrystallized to obtain a white precipitate, the precipitate is filtered and the solid thus obtained is dried at room temperature to give a co-crystal of synthetic tocopherol and L-proline.

35. A health product, food, cosmetics, medicine, pharmaceutic adjuvant or feed, which contains the co-crystal of any one of claims 1-3, 5-8, 10-13, 15-18, 20-23, 25-28 and 30-33.

36. A use of the co-crystal of any one of claims 1-3, 5-8, 10-13, 15-18, 20-23, 25-28 and 30-33 in the preparation of a vitamin nutrition or an antioxidant, and a use of the co-crystal in the preparation of health product, food, cosmetics, medicine, pharmaceutic adjuvant and feed.
